Europäisches Patentamt

European Patent Office

Office européen des brevets

(19)

(11) Publication number: **0 147 682**

A1

# (12) EUROPEAN PATENT APPLICATION

(21) Application number: **84114727.5**

(22) Date of filing: **04.12.84**

(51) Int. Cl.⁴: **A 61 K 37/18**

(30) Priority: **05.12.83 JP 229559/83**

(43) Date of publication of application:
**10.07.85 Bulletin 85/28**

(84) Designated Contracting States:
**AT DE FR GB IT SE**

(71) Applicant: **AJINOMOTO CO., INC.**
**5-8, Kyobashi 1-chome, Chuo-ku**
**Tokyo 104(JP)**

(71) Applicant: **MORISHITA PHARMACEUTICAL CO. LTD.**
**No. 4-29, Doshu-cho Higashi-ku**
**Osaka-shi Osaka-fu(JP)**

(72) Inventor: **Takami, Toru**
**No. 2-20-14, Kurihamadai**
**Yokosuka-shi Kanagawa-ken(JP)**

(72) Inventor: **Okamoto, Hiroo**
**No. 3-306, Higashidanchi Moriyamaekimae**
**321-3, Fuke-cho Moriyama-shi Shiga-ken(JP)**

(72) Inventor: **Okada, Akira No. 102, Mezonkurakuenguchi**
**6-44, Kitatsugi-cho**
**Nishinomiya-shi Hyogo-ken(JP)**

(72) Inventor: **Mori, Shozo**
**No. 7-2, Daishin-cho**
**Morioka-shi Iwate-ken(JP)**

(74) Representative: **Strehl, Schübel-Hopf, Schulz**
**Widenmayerstrasse 17 Postfach 22 03 45**
**D-8000 München 22(DE)**

(54) **Novel amino acid infusion solution.**

(57) A novel amino acid infusion solution contains at least the following amino acids in grams, based on 100 g of the total amount of amino acids stated in the following table :

|  |  |
|---|---|
| 8.5 - 10.0 g | L-Isoleucine |
| 12.0 - 14.0 g | L-Leucine |
| 5.0 - 9.0 g | L-Lysine |
| 3.0 - 5.0 g | L-Methionine |
| 6.0 - 8.0 g | L-Phenylalanine |
| 6.0 - 8.0 g | L-Threonine |
| 1.0 - 2.0 g | L-Tryptophan |
| 8.0 - 16.0 g | L-Valine |
| 0.2 - 0.5 g | L-Tyrosine |
| 5.0 - 10.0 g | L-Alanine |
| 8.0 - 12.0 g | L-Arginine |
| 5.0 - 10.0 g | Glycine |
| 3.0 - 6.0 g | L-Histidine |
| 3.0 - 6.0 g | L-Proline |
| 1.0 - 3.0 g | L-Serine |

EP 0 147 682 A1

EPA-13 968

## NOVEL AMINO ACID INFUSION SOLUTION

The present invention relates to a novel amino acid infusion solution suitable for administration to convalescents, especially postoperative patients.

Heretofore, known amino acid infusion solutions are based on the oral nutritional requirement (for example, (1) an amino acid infusion solution based on necessary amounts for essential amino acids determined by Rose et al, (2) FAO pattern in 1957, (3) amino acid infusion solution based on the composition of amino acids in human milk or whole egg determined by FAO/WHO in 1965, etc.), or are based on the pattern of free amino acids in normal plasma. In practice, amino acid infusion solutions are intravenously administered to postoperative patients so that they are not controlled by the digestive tract and, therefore, it is assumed that the optimum composition would be different from those described above. In recent years, surgical trauma has been further investigated physiologically and biochemically. It has thus been known that the level of free amino acids is greatly distorted particularly in serum and that after an operation a specific aminogram appears in response to catabolism in view of the nutritional physiology. It is therefore desired that the composition of an amino acid infusion solution effective for the postoperative state be a composition which corresponds to the necessary amounts of amino acids whose level has been changed in response to the diseased living body and normalizes the in vivo amino acid pool. Accordingly, the present inventors made extensive investigations with an attempt to develop a novel amino acid infusion solution capable of preventing the catabolism of body proteins and of maintaining the body proteins at a normal level in the state of trauma, using as an index the degree of normalization of the free amino acid pattern in plasma.As a result, the present inventors have accomplished the present invention. During the process of the investigations, the composition of an amino acid infusion solution which can normalize the altered plasma amino acid pattern in the postoperative state was experimentally determined and the composition for use in rats was translated into the composition for use in human, considering differences in plasma amino acid pattern between humans and rats and further considering the similarity regarding the tendency of the change of respective amino acid levels in the postoperative state. Thus the composition "H-5" (Takami et al, Japanese Patent Application OPI 38127/81) was developped. The "H-5" composition was administered to patients with moderate surgical trauma under intravenous hyperalimentation and, the plasma amino acid pattern and the nitrogen balance were compared with the case in which a known amino acid preparation according to Vuj-N formulation was administered. As the result, the degree of normalizing the plasma amino acid pattern in the postoperative state was higher and the balance of nitrogen was better in the case of administering the "H-5" composition, as compared to the Vuj-N formulation. When administering the "H-5" composition, however, an increased level of L-valine and L-tryptophan in the plasma amino acid level and a decreased L-lysine level cause problems which leave room for an improvement. In particular for L-valine a certain correlation has been found between the dose and the concentration in plasma and accordingly, the L-valine dose was modified so as to give a normal level. Further, the range of L-lysine was also examined; it has been found that the degree of normalizing the serum amino acid pattern can be improved and an excellent nutritional effect can be exhibited when the relative proportion of L-valine/L-lysine is kept in the range of 4.0 to 1.1 (as molar ratio).

The amino acid infusion solution of the present invention which has been accomplished based on the above described findings is characterized by comprising at least the following amino acids in the following compositional range :

| Amino Acid | Composition (g/100 g of total amino acids in the table) |
|---|---|
| L-Isoleucine | 8.5 - 10.0 |
| L-Leucine | 12.0 - 14.0 |
| L-Lysine | 5.0 - 9.0 |
| L-Methionine | 3.0 - 5.0 |
| L-Phenylalanine | 6.0 - 8.0 |
| L-Threonine | 6.0 - 8.0 |
| L-Tryptophan | 1.0 - 2.0 |
| L-Valine | 8.0 - 16.0 |
| L-Cystine | 0 - 0.3 |

0147682

| | |
|---|---|
| L-Tyrosine | 0.2 - 0.5 |
| L-Alanine | 5.0 - 10.0 |
| L-Arginine | 8.0 - 12.0 |
| L-Aspartic acid | 0 - 1.0 |
| L-Glutamic acid | 0 - 1.0 |
| Glycine | 5.0 - 10.0 |
| L-Histidine | 3.0 - 6.0 |
| L-Proline | 3.0 - 6.0 |
| L-Serine | 1.0 - 3.0 |

In case one or more of the above stated optional components L-Cystine, L-Aspartic acid and L-Glutamic acid is or are present, the preferred lower limit for the amount of these optional components is 0.01 g per 100 g of total amino acids of the composition.

In the amino acid infusion solutions having the stated composition L-Cystine may be partially or wholly replaced by cysteine. Further methionine and/or tyrosine may be partially or wholly replaced by phenyl alanine.

The amino acid infusion solution of the present invention has a pH ranging from 5.0 to 7.8, preferably 6.5 to 7.5. As a pH controlling agent, organic acids, for example, acetic acid, malic acid, etc. may be used. The concentration of the amino acids is not particularly limited but determined depending upon the specific use , for example, for uses, such as infusion solution in the central vein, infusion solution in the peripheral blood vessel, etc. It is, however, preferred that the concentration be approximately 5 to 12 %.

The amino acids used in the present invention may be used either as free acids or in the form of a salt, such as metals salts, e.g., sodium salt, potassium salt, etc.; mineral acid salts, e.g., sulfate, hydrochloride, etc.; organic acid salts, e.g., malate, etc.

Further the amino acid infusion solution of the present invention may additionally contain electrolytes, vitamins and other additives known for conventional amino acid infusion solutions. The amino acid infusion solution may further contain stabilizers, pH controlling agents, etc. required for preparation of the infusion solution.

The amino acid infusion solution of the present invention may be prepared without any difficulty and any process for preparing known amino acid infusion solutions is applicable.

Hereafter, the present invention will be described more in detail, referring to the following examples.

Example 1

Each of the amino acids is dissolved in distilled water for use in injection as shown the table below. After the pH of the resulting aqueous solution of the amino acids is adjusted to about neutrality, bacteriae are removed by filtration. Then, the solution is charged in a vial or a plastic bag. After substituting the air with nitrogen, the vial or bottle is plugged and sterilized by heating in a conventional manner to prepare the intended amino acid infusion solution.

| Amino Acid | g/l |
| --- | --- |
| L-Isoleucine | 9.34 |
| L-Leucine | 12.43 |
| L-Valine | 14.90 |
| L-Lysine acetate | 10.16 |
| L-Methionine | 4.45 |
| L-Phenylalanine | 7.07 |
| L-Tyrosine | 0.37 |

| | |
|---|---|
| L-Threonine | 7.53 |
| L-Tryptophan | 1.30 |
| L-Arginine | 8.62 |
| L-Histidine | 4.88 |
| Glycine | 7.85 |
| L-Alanine | 6.51 |
| L-Serine | 1.69 |
| L-Glutamic acid | 0.48 |
| L-Aspartic acid | 0.91 |
| L-Proline | 4.48 |
| L-Cysteine | 0 |

| | |
|---|---|
| total amount of amino acids in the free form | 100.0 |

## Example 2

Each of the amino acids shown in the following table is treated in a manner similar to Example 1 to obtain the aimed amino acid infusion solution.

| Amino Acid | g/l |
|---|---|
| L-Isoleucine | 9.1 |
| L-Leucine | 12.90 |
| L-Valine | 14.00 |
| L-Lysine acetate | 10.01 |
| L-Methionine | 4.40 |
| L-Phenylalanine | 7.00 |
| L-Tyrosine | 0.40 |
| L-Threonine | 7.50 |
| L-Tryptophan | 1.30 |
| L-Arginine | 9.00 |
| L-Histidine | 5.00 |
| Glycine | 7.00 |
| L-Alanine | 7.10 |
| L-Serine | 1.70 |
| L-Glutamic acid | 0.50 |

| | |
|---|---|
| L-Aspartic acid | 1.00 |
| L-Proline | 5.00 |
| L-Cystine | 0 |

| | |
|---|---|
| total amounts of amino acids in the free form | 100.0 |

## Example 3

Each of the amino acids shown in the following table is treated in a manner similar to Example 1 to obtain the aimed amino acid infusion solution.

| Amino Acid | g/l |
|---|---|
| L-Isoleucine | 9.20 |
| L-Leucine | 13.50 |
| L-Valine | 10.00 |
| L-Lysine acetate | 10.20 |
| L-Methionine | 4.40 |
| L-Phenylalanine | 7.00 |
| L-Tyrosine | 0.40 |
| L-Threonine | 6.50 |
| L-Tryptophan | 1.30 |
| L-Arginine | 11.50 |
| L-Histidine | 5.00 |
| Glycine | 5.00 |
| L-Alanine | 7.15 |
| L-Serine | 1.70 |
| L-Glutamic acid | 0.50 |
| L-Aspartic acid | 1.00 |
| L-Proline | 5.00 |
| L-Cystine | 0.05 |

| | |
|---|---|
| total amount of amino acids in the free form | 100.0 |

Test Example 1

A high calory infusion solution (daily dose : 40 ml/kg body weight/day containing 13.2 ml of the amino acid infusion solution obtained in Example 1, 6 g of glucose and, vitamins, minerals and the like) was administered to patients in the postoperative state with medium to severe trauma (for example, gastrectomy, cholecystoectomy) for consective 7 days. For the purpose of comparison, an amino acid infusion solution having the "H-5" composition (composition shown in Comparative Example 1) and a commercially available amino acid infusion solution (composition shown in Comparative Example 2) were used and evaluated in a manner similar to this Example 1.

Comparative Example 1

| Amino Acid | g/l |
|---|---|
| L-Isoleucine | 8.88 |
| L-Leucine | 12.43 |
| L-Valine | 18.71 |
| L-Lysine acetate | 5.66 |
| L-Methionine | 4.45 |
| L-Phenylalanine | 8.20 |
| L-Tyrosine | 0.37 |
| L-Threonine | 7.53 |
| L-Tryptophan | 2.85 |
| L-Arginine | 7.15 |
| L-Histidine | 4.88 |
| Glycine | 7.10 |
| L-Alanine | 5.58 |
| L-Serine | 1.19 |
| L-Glutamic acid | 0.48 |

- 9 -

0147682

| | |
|---|---|
| L-Aspartic acid | 0.71 |
| L-Proline | 4.48 |
| L-Cysteine | 1.00 |
| total amount of amino acids in the free form | 100.0 |

As the result, it is observed that, as shown in Table 1, the composition of Example 1 is effective in terms of improvement of nitrogen balance and prevention of decrease in body weight, as compared to any of the comparative examples and, the degree of normalizing the plasma amino acid pattern except for branched amino acids is higher than in the comparative examples.

Comparative Example 2

| Amino Acid | g/l |
|---|---|
| L-Isoleucine | 5.60 |
| L-Leucine | 12.50 |
| L-Valine | 4.50 |
| L-Lysine acetate | 12.41 |
| L-Methionine | 3.50 |
| L-Phenylalanine | 9.35 |
| L-Tyrosine | 0.35 |
| L-Threonine | 6.50 |
| L-Tryptophan | 1.30 |
| L-Arginine | 7.90 |
| L-Histidine | 6.00 |
| Glycine | 10.70 |
| L-Alanine | 6.20 |
| L-Serine | 2.20 |
| L-Glutamic Acid | 6.50 |

| | |
|---|---|
| L-Aspartic acid | 3.80 |
| L-Proline | 3.30 |
| L-Cysteine | 1.00 |
| total amount of amino acids in the free form | 100.00 |

TABLE 1

| | Example 1 | Comparative Example 1 | Comparative Example 2 |
|---|---|---|---|
| Rate of decrease in body weight (%/7 days) | -2.64 | - | -3.71 |
| Cumulative nitrogen balance (gN/7 days) | +4.31* | -1.25 | -7.67 |
| Plasma amino acid pattern | | | |
|    normalization degree [1] (2 days after operation) | 0.95 | 0.93 | 0.92 |

\* p < 0.01 vs Comparative Example 2

1) Tamura et al., "Nutrition and Food", vol. 22, 494 - 496 (1969)

In particular, the degree of normalizing the serum amino acid pattern is higher in Example 1 than in Comparative Example 1 and, an improved effect is noted in Example 1.

Test Example 2

A high calory infusion solution (daily dose : 50 ml/kg body weight/day containing 16.5 ml of the amino acid infusion solution obtained in Example 1, 6 g of glucose and, vitamins, minerals, and the like) was administered to postoperative patients with severe trauma (for example, esophagectomy, gastrectomy) for consecutive 7 days.

For the purpose of comparison, a commercially available amino acid infusion solution (composition shown in Comparative Example 2) were used and evaluated in a manner similar to Example 1.

### TABLE 2

|  | Example 1 | | | Comparative Example 2 | | |
|---|---|---|---|---|---|---|
| Postoperative day | 3 | 5 | 7 | 3 | 5 | 7 |
| Retinol binding protein | 66.7 % | 88.9 % | 118.5 % | 40.5 % | 64.9 % | 83.8 % |
| Prealbumin | 58.6 % | 89.0 % | 117.3 % | 46.3 % | 60.0 % | 73.3 % |

Preoperative value = 100 % (n = 5 - 6)

As the result, it is observed that, as shown in Table 2, the composition of Example 1 is effective in terms of improvement of rapid recovery after the operation of rapid turnover protein (Retinol Binding Protein and Prealbumin) in the plasma, as compared to any of the comparative examples and promotes metabolism of body protein in the postoperative patients.

0147682

CLAIMS

1. An amino acid infusion solution, containing at least the following amino acids in g per 100 g of the total amount of amino acids :

|        |   |        |   |                 |
|--------|---|--------|---|-----------------|
|  8.5   | - | 10.0 g | L-Isoleucine    |
| 12.0   | - | 14.0 g | L-Leucine       |
|  5.0   | -  | 9.0 g | L-Lysine        |
|  3.0   | -  | 5.0 g | L-Methionine    |
|  6.0   | -  | 8.0 g | L-Phenylalanine |
|  6.0   | -  | 8.0 g | L-Threonine     |
|  1.0   | -  | 2.0 g | L-Tryptophan    |
|  8.0   | - | 16.0 g | L-Valine        |
|  0.2   | -  | 0.5 g | L-Tyrosine      |
|  5.0   | - | 10.0 g | L-Alanine       |
|  8.0   | - | 12.0 g | L-Arginine      |
|  5.0   | - | 10.0 g | Glycine         |
|  3.0   | -  | 6.0 g | L-Histidine     |
|  3.0   | -  | 6.0 g | L-Proline       |
|  1.0   | -  | 3.0 g | L-Serine        |

with the provision that tyrosine may be partially or wholly replaced by phenylalanine.

2. Amino acid infusion solution according to claim 1, wherein the molar ratio of L-valine/L-lysine is in the range of 4.0 to 1.1.

3. Amino acid infusion solution according to claim 1 or 2, which contains usual additives.

4. An amino acid infusion solution according to claim 1, containing additionally at least one of the following amino acids in g per 100 g of the total amount of amino
acids :

    0.01 - 0.3 g L-Cystine
    0.01 - 1.0 g L-Aspartic acid
    0.01 - 1.0 g L-Glutamic acid

with the provision that cystine may be partially or wholly
replaced by cysteine and/or methionine and tyrosine may be
partially or wholly replaced by phenylalanine.

0147682

1. A process for producing an amino acid infusion solution, characterized in that the following amino acids, indicated in g per 100 g of the total amount of amino acids, are dissolved in distilled water for use in injection either in the free form or in the form of a salt :

8.5 - 10.0 g L-Isoleucine
12.0 - 14.0 g L-Leucine
5.0 - 9.0 g L-Lysine
3.0 - 5.0 g L-Methionine
6.0 - 8.0 g L-Phenylalanine
6.0 - 8.0 g L-Threonine
1.0 - 2.0 g L-Tryptophan
8.0 - 16.0 g L-Valine
0.2 - 0.5 g L-Tyrosine
5.0 - 10.0 g L-Alanine
8.0 - 12.0 g L-Arginine
5.0 - 10.0 g Glycine
3.0 - 6.0 g L-Histidine
3.0 - 6.0 g L-Proline
1.0 - 3.0 g L-Serine

with the provision that tyrosine may be partially or wholly replaced by phenylalanine,
and the pH of the solution is adjusted to a value in the range of 5.0 to 7.8.

2. A process according to claim 1, wherein the amino acids are dissolved in distilled water in a concentration of 5 to 12 % by weight.

3. A process according to claim 1 or 2, wherein the molar ratio of L-valine/L-lysine is in the range of 4.0 to 1.1.

4.  A process according to any of the claims 1 to 3, in which usual additives are added to the solution.

5.  A process according to any of the claims 1 to 4, wherein additionally at least one of the following amino acids, in g per 100 g of the total amount of amino acids :

           0.01 - 0.3 g L-Cystine
           0.01 - 1.0 g L-Aspartic acid
           0.01 - 1.0 g L-Glutamic acid

with the provision that cystine may be partially or wholly replaced by cysteine and/or methionine and tyrosine may be partially or wholly replaced by phenylalanine, is  dissolved in the solution.

**European Patent Office**

# EUROPEAN SEARCH REPORT

EP 84 11 4727

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | DE-A-2 946 563 (B. BRAUN MELSUNGEN AG) <br> * Pages 1-2, claims 1-2 * | 1-4 | A 61 K 37/18 |
| X | FR-A-2 365 552 (DR. EDUARD FRESENIUS CHEMISCH-PHARMAZEUTISCHE INDUSTRIE KG) <br> * Pages 7-8, claims 1-8 * | 1-4 | |
| X | WO-A-8 303 969 (THE TRUSTEES OF COLUMBIA UNIVERSITY IN THE CITY OF NEW YORK) <br> * Page 40, line 1 - page 41, line 21, claims 1-2 * | 1-4 | |
| A | US-A-2 662 046 (EUGENE E. HOWE) <br> * Column 5, lines 14-25, claim 1 * | 1-4 | TECHNICAL FIELDS SEARCHED (Int. Cl.4) |
| A | FR-A-2 435 247 (AJINOMOTO & CO. INC.) <br> * Page 15, lines 1-26, claim * | 1-4 | A 61 K |

The present search report has been drawn up for all claims

| Place of search | Date of completion of the search | Examiner |
|---|---|---|
| THE HAGUE | 08-03-1985 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82